# EUROPEAN PATENT APPLICATION

(11) **EP 4 094 738 A1**
(43) Date of publication of application: **30.11.2022**
(21) Application number: 21175959.2
(22) Date of filing: 26.05.2021
(51) Int. Cl.: A61F 13/42

(54) **SYSTEM AND METHOD FOR DETECTING AN INCONTINENCE EVENT IN AN ABSORBENT ARTICLE**

(71) Applicant: AssistMe GmbH, 10555 Berlin (DE)
(72) Inventor: Fraine, Jonathan, 13467 Berlin (DE)
(74) Representative: Gulde & Partner

(57) **Abstract**

The present disclosure concerns a method for detecting an incontinence event in an absorbent article (401), the method comprising the steps of: receiving (S101) a signal from a capacitive moisture sensor, CMS, (201) attached to the absorbent article (401), the signal being dependent on presence of liquid in the absorbent article (401); determining (S102) based on the signal and a predefined reporting configuration comprising a reference value associated with the signal, whether to report an incontinence event; and reporting (S103) an incontinence event based on the determination (S102). A corresponding system is also disclosed.

## Description

### Field of the Invention

The present invention relates to a system and a method for detecting an incontinence event in an absorbent article, particularly a system with automatic detection and reporting of an incontinence event that results in a need of replacement of an absorbent article. The invention further relates to limiting a number of instances of possibly reporting of (a) false-positive incontinence event(s).

### Technological Background

One of the biggest challenges that modern societies might face in the coming years is the aging of their populations. The number of caregivers and elderly at nursing homes will become less balanced, therefore less caregivers will attend more residents.

Already today caregivers do check on residents according to a tight time schedule, not only making it an exhausting workload but also rendering it difficult to figure out, which urgent need to attend first. Also, important to note is that caregivers have assigned a certain number of residents they have to monitor on each shift, which renders efficient scheduling even more important.

Incontinence is a medical condition, in which there is uncontrolled release of natural discharges or evacuations from the bladder and/or bowel. Urinary incontinence refers to loss of bladder control resulting in involuntary or uncontrolled urination. While some forms of incontinence, particularly urinary/bladder incontinence are relatively widespread, the condition typically affects the elderly and the infirm and is more prevalent among women.

In the past, to comply with regulations and protocols and to ensure that incontinence sufferers in care institutions such as hospitals, nursing homes, aged care facilities and geriatric institutions are appropriately cared for, it has been necessary for staff to manually check these patients on a regular basis. Apart from the unpleasantness involved with manual checks, such a regimen also places a strain on staff resources. Manually checking for wetness can also cause interruption to a patient's rest and sleep.

Incontinence care involves use of products such as diapers, feminine hygiene products, and adult incontinence aids. These products may be called sanitary absorbent items (articles) or absorbent items (articles) in general. Such absorbent articles may comprise superabsorbent polymers (SAPs) that can absorb and retain liquid. In particular, SAPs are capable of retaining large amounts of a liquid relative to their own mass. It is thus frequently the case that modern absorbent articles absorb more than a single urination and may be used for longer periods of time. In the context of the present disclosure, an incontinence event is related to a change of an amount of liquid in an absorbent article. In turn such change may lead to leaking of the absorbent article or may lead to approaching a state close to leaking of the absorbent article.

However, known absorbent articles still suffer from the fact that a manual check is required relatively frequently. Therefore, the aim of the present invention is to avoid tactile, and visual check of the patient and to reduce time spent overseeing the absorbent articles as well as allowing to utilize capabilities of the absorbent articles to their full extent. This in turn achieves the aim of significantly increasing comfort of incontinence-affected patients.

### Description of the Invention

The objective of the invention is solved and the disadvantages of the prior art are overcome by the subject-matter of the present invention, i.e., by a system and a method for detecting an incontinence event in an absorbent article according to the appended claims.

A wide spread miniaturization of electronic devices such as sensors and their increasing power efficiency allows to monitor a variety of items, for example objects as well as persons. Different sensors are used to provide sensed data to receivers such as so-called smart devices.

An aspect of the invention relates to a method for detecting an incontinence event in an absorbent article, the method comprising the steps of: receiving a signal from a capacitive moisture sensor, CMS, attached to the absorbent article, the signal being dependent on the presence of liquid in an absorbent article; determining based on the signal and a predefined reporting configuration comprising a reference value associated with the signal, whether to report an incontinence event; and reporting an incontinence event based on the determination.

The CMS is configured to induce an electrical signal to the absorbent article, and to measure a capacitance of the absorbent article and a liquid content thereof. The capacitance measurement can be translated to a digital signal output that can be subsequently received and analyzed for detecting incontinence events and providing notifications to a caregiver (e.g., via a smartphone application).

In a preferred embodiment the inducer of the electrical signal may be a separate entity from the CMS entity.

In this context, attaching the CMS to an absorbent article may not only concern attaching the CMS to the external side of the absorbent article but also mean positioning the CMS within the article i.e. embedding a CMS within the article. Further, the CMS may also be fully or partially positioned within the absorbent article. To this end fastening or mounting techniques such as gluing, stitching, or similar procedures may be employed.

The electrical signal detected by the CMS may not only be dependent on the presence of a liquid as such in the absorbent article but is preferably dependent on the amount of the liquid that is present in the absorbent article.

According to the present disclosure, the predefined reporting configuration comprises a reference value that is associated with the signal and which may be as simple as a signal value such as an average value or a maximum value at which a given alert is to be communicated/reported. However, there may be present different reporting conditions within the reporting configuration. Such conditions may be correlated with reporting different levels of remaining capacity of the absorbent article. For example, reports may be given at a step of 5% from 100% to 0% absorption before leakage. Each such remaining capacity may be associated with an expected signal from the CMS. Additionally, alarms may be raised at other thresholds of remaining capacity.

Such expected signals will typically be dependent on the absorbent article and thus a configuration step will preferably involve a process of testing a given absorbent article in order to define its associated reporting configuration.

For example, an absorbent article is tested for its maximum capacity by submerging it in a tank of liquid and measuring the maximum liquid intake. Additionally, there may also be measured an operational capacity by introducing liquid (in a manner close to real use-case situation) into the article and detecting leakage at a point of maximum operational capacity. The maximum operational capacity may be different than the maximum capacity. The maximum operational capacity is thereafter correlated with a specific sensor signal response. A reference signal value for an empty absorbent article is also determined. Correspondingly, signal values at different remaining capacity levels may be read and stored for reference. Alternatively, it may be assumed that the signal obtained from the sensor changes in a relatively linear fashion from the set first value indicating an empty absorbent article to the set second value indicating a fullness at operational capacity of the absorbent article. When a systematic approach is defined, the respective thresholds may be determined automatically.

Such tested values are made available as the predefined reporting configuration, which is used by the incontinence detection method. Such predefined reporting configuration may be stored in a local memory of a system executing the method.

In a preferred embodiment, the method further comprises the steps of: receiving at least one supplementary signal from one or more additional sensors and determining, based on the signal, the at least one supplementary signal, and the predefined reporting configuration further comprising a reference value associated with the at least one supplementary signal, whether to report the incontinence event.

In this context the one or more additional sensors may be attached to an absorbent article but may also or alternatively be monitoring the patient (e.g. sensors attached to the patient or worn by the patient).

The additional sensors provide further insights in order to better classify specific medical and non-medical conditions, through two or more different measurement devices (e.g. an accelerometer, a moisture sensor, a temperature sensor).

In addition, or alternatively, additional sensors may be present on/in the absorbent article. Such additional sensors may be further moisture sensors and/or further temperature sensors that may be positioned in different zones of the absorbent article. For example, when a diaper is considered comprising three zones, one or more additional sensor(s) may be present in a crotch area, one or more additional sensor(s) may be present in an abdomen area, one or more additional sensor(s) may be present in a back area (e.g. close to the lumbar area of the back).

Such additional sensors may be installed inside a wearable device used by subjects suffering from incontinence condition, pressure ulcers, and orientation deficiencies in a care home environment.

Inside such a wearable, the present system includes one or more additional sensor(s). Each of these additional sensors may be introduced to identify particular conditions. By combining these additional sensors (and readings of their output signals) into a combined analysis, the system is able to identify meaningful outliers in the life cycle of care home residents. Examples include dropping water on the abdomen (cold and wet), sitting next to a furnace or a radiator (warm and dry), etc. Identifying these situations improves accuracy in both diagnosing/detecting/recognizing a patient's medical events; but also, in creating a resident profile per user, such as their urination pattern, fever conditions, or sleep patterns.

For example, a principle false-positive detection in the present system is a non-urine liquid being deposited into an absorbent article. To reduce false positive detections, the system may treat data from individual sensors as unique attributes. The additional data may be examined to determine specific medical conditions and/or business logic triggers. In practice, several of these measurements may be combined as independent measurements, which can determine more specific information than generic information obtained from a single CMS.

As an example, data streams for the thermometer and moisture sensor may be considered together to determine more than just urine content of the absorbent article, but also non-urine fluids or non-fluid causes of the triggering mechanisms. Examples given above were dropping water on the abdomen (wet and cold), sitting next to a furnace/radiator (dry and warm). A third example could include dropping tea on the abdomen, which would generate a 'warm+wet' signal, similar to a urination pattern; but given that there may be a plurality of thermometers positioned in different zones of the absorbent article (e.g. 8 thermometers), it may be determined where the source of the liquid has come from (e.g. crotch region, abdomen region, back region).

Because tea and urine contain different molecules, it is possible to measure a different capacitance (using the electronic signal for volume of moisture according to the present invention) caused by the tea than caused by urine. Therefore, the reporting configuration may also consider a type of detected liquid based on the measured capacitance.

In that regard, the predefined reporting configuration may further comprise a reference value associated with the at least one supplementary signal. For example, the abdomen temperature sensor will be considered to report cold below 30 degrees Celsius and hot above 40 degrees Celsius, which may be combined with a moisture sensor to detect spilling of a hot or cold liquid at the abdomen area.

For example, if a resident drops tea on their abdomen, the diaper region near their genitalia will continue to have space for more urine. Missing this determination (i.e., dropping tea) would flag the diaper as full before it is necessary to change it, thus reducing the efficiency of care givers using the present invention.

Therefore, in a more general approach, in order to reduce false-positive events, gating sensors may be used to identify events and circumstances which could affect the interpretation of the main sensor measurements. Signals reported by the gating sensors are analyzed together with the signals received from the main CMS.

Additionally, and/or alternatively, in order to increase the confidence level of detection by the main CMS, confirmation sensors can be used. The confirmation sensors may be one or more further CMS(s).

The one or more confirmation sensors are used in addition to the main CMS for a parallel detection of incontinence events, such that an event is considered detected when detected by the main CMS and at least one confirmation sensor.

For example, different gating sensors and/or confirmation sensors could be associated with different zones of an absorbent article.

In an additional preferred variant the at least one supplementary signal comprises a capacitance measurement of the absorbent article and/or wherein the at least one supplementary signal is selected from a group comprising: a temperature signal, a humidity signal, an acceleration signal, a gas presence signal, a motion signal, an electromagnetic signal, a chemical composition detection signal.

Each such supplementary signal monitored may provide data for analysis and interpretation in combination with analysis and interpretation of data provided by the CMS.

For example, the absence/presence of a specific chemical composition may exclude or confirm a urine presence in the absorbent article. Also, the absence/presence of a specific infra-red reflective signal measured by an optical sensor may exclude or confirm urine presence.

Features disclosed with respect to this embodiment may be combined with features of the two previously presented embodiments.

In an additional preferred variant, the predefined reporting configuration indicates a remaining capacity threshold of the absorbent article.

As explained above, the signal read from the CMS and/or the one or more additional sensors may be indicative of the estimated absorbent capacity that is left, i.e., 10%, 20% of the full capacity. Therein, the estimated remaining capacity might be based on reference data. Such estimation based on reference data may be made after receiving raw data signals (e.g. not processed to infer meaning) from the respective sensors or be made by the CMS itself.

It is beneficial to report to caretakers clear information and thus a preferred way of informing a caretaker is to give information that, for example, a given absorbent article has 40% capacity left (or any other predefined remaining capacity threshold). In addition, information may be given for how long the given absorbent article has been in use e.g. for 4 hours. Further, it may be recorded how capacity of a given absorbent article associated with a given CMS changed over time. It may further be preferred to give information on a predicted remaining capacity and/or remaining time. To this end the monitoring system may comprise a timer, which may be reset.

Features disclosed with respect to this embodiment may be combined with features of the preceding embodiments.

In an additional preferred variant, the CMS is configured to detect the signal by performing a fringe capacitance measurement.

A capacitive sensor converts a change in position or properties of dielectric materials into an electrical signal. Specifically, a fringe field capacitor was designed for various applications like chemical level detection, water level detection, or soil moisture measurement.

To detect incontinence, the present invention is preferably configured to measure a fringe capacitance related to the monitored absorbent article. The fringe capacitance induces an electric field between electrodes, such as interdigitated electrodes (IDE) over an array. The capacitance between the electrodes ("fingers") dominates the signal, but does not change over time. As the electric field passes away from the electrodes, through the absorbent article (or other mediums), bends, and penetrates back into the next electrodes, the capacitance associated with this electric field is referred to as "fringe capacitance". As liquid is introduced into the monitored absorbent article, the fringe capacitance is affected by the change in the permittivity of the electric constant through which the fringe electric field is passing. This change in fringe capacitance is the determining signal for the presence of liquid in the monitored absorbent article.

Features disclosed with respect to this embodiment may be combined with features of the preceding embodiments.

In an additional preferred variant, the CMS is configured to detect the signal by detecting a frequency dependent capacitance at a plurality of frequencies.

A plurality of frequencies is a preferred choice in order to obtain a series of measurements that first of all may be correlated and secondly may allow detecting different liquids.

As indicated earlier, different liquids comprise different molecules. For example, due to the fact that tea and urine contain different molecules, it is possible to measure a different capacitance for tea and urine. Similarly, other liquids may be considered, which may require measurements at different frequencies.

In a preferred embodiment, the plurality of frequencies are spaced apart by 10 kHz, and/or 1 kHz, and/or 100 Hz, and/or 10 Hz.

Features disclosed with respect to this embodiment may be combined with features of the preceding embodiments.

In an additional preferred variant, the frequency dependent capacitance is detected at the plurality of frequencies in a range of 100Hz to 100 kHz and/or in a range of 10 kHz to 100 kHz and/or in a range of 40 kHz to 80 kHz.

The inventors have found that detection of the frequency dependent capacitance is preferably executed at frequencies in a range of 100Hz to 100 kHz which may be further narrowed to a subrange of 10 kHz to 100 kHz which may be further narrowed to a subrange of 40 kHz to 80 kHz.

In general, lower frequencies as the aforementioned are less preferred because they introduce noise and a response is dominated by resistivity while higher frequencies as the aforementioned are also less preferred because the responses tend to be non-linear and are capacitance dominated.

In particular, frequencies lower than 40kHz are less beneficial because they introduce noise and a response is dominated by resistivity while frequencies above 80kHz tend to be non-linear in terms of responses and are capacitance dominated.

Further, frequencies between 40 kHz to 80 kHz are relatively linear in terms of responses to urine presence and are therefore most preferred.

Using a curvature analysis and/or knee detection algorithm (i.e. temporally relative curvature measurement), it is possible to robustly identify the location of (a) urination event(s) as discontinuities in the time series. Because curvature detection algorithms are designed under the assumption of continuous and differential time-series data, the change of state from "dry to wet" during a first incontinence event -- and the change of state from "relaxing" to "urinating" during later incontinence events -- introduce discontinuities and non-differentiable behaviour. These discontinuities and non-differentiable behaviour produce temporally significant and correlated outliers in the resulting curvature and/or knee detection algorithm(s) that provide identifiable, and classifiable, signals using for example temporal machine learning algorithms, such as an ensemble of gradient boosted trees, and/or Gaussian processes, and/or deep learning (neural) networks.

Features disclosed with respect to this embodiment may be combined with features of the preceding embodiment.

It is additionally preferred that the determining, whether to report the incontinence event, further comprises performing a cross-comparative and/or cross-correlative analysis of the capacitance detected at the plurality of frequencies.

As already explained above, a plurality of measurements is obtained from the CMS while in a preferred embodiment, the frequencies of the plurality of frequencies are spaced apart by 10 kHz, and/or 1 kHz, and/or 100 Hz, and/or 10 Hz. Alternatively, the frequencies may be spaced by 10 kHz, which will result in fewer measurements and less time required for signal processing. A drawback will be such that less data is available for comparison and/or correlation at larger spacings.

Features disclosed with respect to this embodiment may be combined with features of the preceding embodiments related to the plurality of frequencies.

It is additionally preferred that the method further comprising a step of communicating the reported incontinence event to an external device.

The external device may be a server of a mobile device such as a tablet or a smartphone running a software application communicating with the system according to the present invention. The communication is preferably wireless over a communication link such as Wi-Fi or Bluetooth or ZigBee or the like.

Features disclosed with respect to this embodiment may be combined with features of the preceding embodiments.

Another aspect of the present invention is a system for detecting incontinence events in an absorbent article, the system comprising: a capacitive moisture sensor, CMS, attached to the absorbent article, the CMS being configured to induce an electrical stimulus signal to the absorbent article and to determine a signal by a capacitance measurement, wherein the signal is dependent on a presence of liquid in the absorbent article; and a controller configured to receive a data signal from the CMS; the controller being further configured to execute the steps of the method according to the first aspect of the present invention.

The system implements the method according to the first aspect of the present invention by employing a controller. The system may be realized using dedicated components or custom made FPGA or ASIC circuits. The system comprises necessary common components such as a data bus communicatively coupled to a memory or a power supply such as a battery. Additionally, other components of the system are communicatively coupled to the system bus so that they may be managed by the controller. Such common modules of computer systems are typical in the technical field concerned as will be evident to one skilled in the art. For the sake of brevity, details of such common modules of computer systems are omitted herein.

The system may for example comprise a communication module allowing for reception of configuration data and/or transmission of collected data as well as detected events.

It is additionally preferred that the CMS is configured to detect fringe capacitance.

It is additionally preferred that the system further comprises one or more additional sensors configured to provide at least one supplementary signal; wherein the controller is further configured to execute the steps of the method according to the present invention where such additional sensors are used.

The system according to the present invention may be embedded in a strip-like or band-like article that may be positioned on or in the monitored absorbent article by having its first end positioned in proximity to the abdomen area (in particular the pelvic region) and having its second end in proximity to the lumbar area whereas the length of the strip/band runs along the crotch area.

Such a strip/band may have different lengths and or widths depending on the monitored absorbent article.

Different additional sensors may be positioned along the length of the strip/band. Such additional sensors may be gating or confirmation sensors as explained above.

In other optional embodiments, the strip-like or band-like shape may be different and for example comprise more than one strip connected to each other at different angles. The different strips may coincide with different zones of the monitored absorbent article.

Features disclosed with respect to this embodiment may be combined with features of the preceding embodiments.

It is additionally preferred that the controller is further configured to: transmit data received from the CMS and/or the additional sensors to an external server for determining whether to report the incontinence event; and receive from the external server a result of the determination.

In this embodiment a client-server arrangement is considered, wherein a controller of an absorbent article may be a light-weight system having relatively low data processing capabilities. In such a case, data received from the CMS and/or the additional sensors may be transferred via a communication link to an external server that will process the data and determine whether to report the incontinence event.

Features disclosed with respect to this embodiment may be combined with features of the preceding embodiments.

It is additionally preferred that the one or more additional sensors comprise: gating sensors, particularly selected from a group comprising: a temperature sensor, a humidity sensor, an acceleration sensor, a gas sensor, a motion sensor; and/or at least one confirmation sensor, particularly at least one CMS attached to the absorbent article.

Use of such additional gating and/or confirmation sensors has been explained above when describing features of the present method.

Another aspect of the present invention is a computer program comprising program code means for performing all the steps of the computer-implemented method according to the present invention when said program is run on a computer.

Another aspect of the present invention is a computer readable medium storing computer-executable instructions performing all the steps of the computer-implemented method according to the present invention when executed on a computer.

Such computer programs are typically executed by utilizing the computing resources in a computing device. Applications are stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein.

Yet another aspect of the present invention is an absorbent article comprising the system according to the present invention.

Typically, an absorbent article may be manufactured as including the system according to the present invention. This elevates a burden of properly placing the system on/in the absorbent article from caretakers. However, in other embodiments, a caretaker may equip an off-the-shelf absorbent article with the system. In such a case, the controller of the system must be programmed with the predefined reporting configuration tailored for this specific absorbent article. Such a configuration may be made available to users via a database and/or an online tool or a software application for mobile devices.

Also, a person of skill in the art should recognize that the functionality of various computing devices may be combined or integrated into a single computing device, or the functionality of a particular computing device may be distributed across one or more other computing devices without departing from the scope of the embodiments of the present invention.

Further aspects of the present invention are disclosed in the dependent claims or the following description of the drawings.

### Brief Description of the Drawings

Features will become apparent to those of ordinary skill in the art by describing in detail exemplary embodiments with reference to the attached drawings in which:
- Figure 1: illustrates a schematic drawing of a method according to the present invention;
- Figure 2: presents a schematic drawing of application of gating sensors according to the present invention;
- Figure 3: shows a schematic drawing of a use case of a main sensor and a temperature sensor according to the present invention;
- Figures 4A and 4B: illustrate an absorbent article comprising the system according to the present invention in two exemplary embodiments;
- Figure 5: illustrates a schematic drawing of combining data from sensors in two or more measurement dimensions; and
- Figure 6: shows a block diagram of the system according to the present invention.

### Detailed Description of the Invention

Reference will now be made in detail to embodiments, examples of which are illustrated in the accompanying drawings. Effects and features of the exemplary embodiments, and implementation methods thereof will be described with reference to the accompanying drawings. In the drawings, like reference numerals denote like elements, and redundant descriptions are omitted. The present invention, however, may be embodied in various different forms, and should not be construed as being limited to only the illustrated embodiments herein. Rather, these embodiments are provided as examples so that this disclosure will be thorough and complete, and will fully convey the aspects and features of the present invention to those skilled in the art.

Accordingly, processes, elements, and techniques that are not considered necessary to those having ordinary skill in the art for a complete understanding of the aspects and features of the present invention may not be described. In the drawings, the relative sizes of elements, layers, and regions may be exaggerated for clarity.

As used herein, the term "and/or" includes any and all combinations of one or more of the associated listed items. Further, the use of "may" when describing embodiments of the present invention refers to "one or more embodiments of the present invention." In the following description of embodiments of the present invention, the terms of a singular form may include plural forms unless the context clearly indicates otherwise.

It will be understood that although the terms "first" and "second" are used to describe various elements, these elements should not be limited by these terms. These terms are only used to distinguish one element from another element. For example, a first element may be named a second element and, similarly, a second element may be named a first element, without departing from the scope of the present invention. Expressions such as "at least one of," when preceding a list of elements, modify the entire list of elements and do not modify the individual elements of the list.

As used herein, the term "substantially," "about," and similar terms are used as terms of approximation and not as terms of degree, and are intended to account for the inherent deviations in measured or calculated values that would be recognized by those of ordinary skill in the art. Further, if the term "substantially" is used in combination with a feature that could be expressed using a numeric value, the term "substantially" denotes a range of +/- 5% of the value centered on the value.

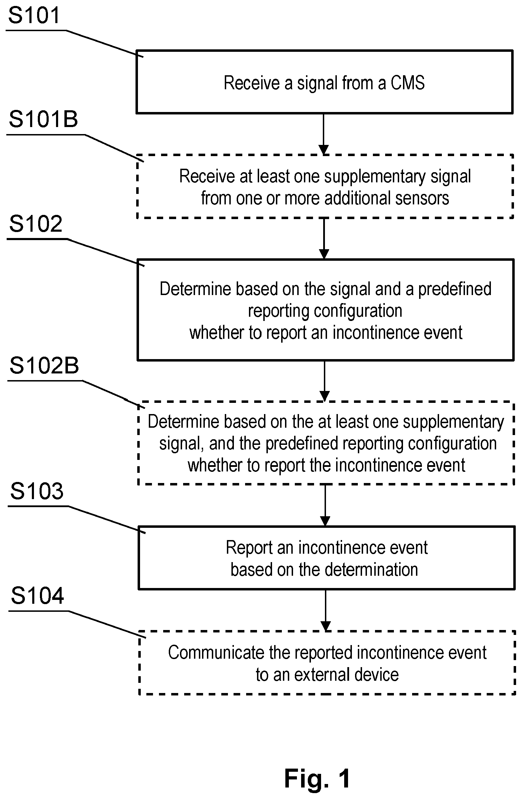

Fig. 1 illustrates a schematic drawing of a method according to the present invention. The first step of the method S101 is receiving a signal from a capacitive moisture sensor, CMS, attached to (the CMS may also be fully or partially positioned in the absorbent article) an absorbent article, the signal being dependent on the presence of liquid in the absorbent article. Next the process executes step S102 concerning determining whether to report an incontinence event based on the signal and a predefined reporting configuration comprising a reference value associated with the signal and subsequently the step S103 of reporting an incontinence event based on the determination.

In a particularly preferred embodiment, an optional step S101B and an optional step S102B are present, wherein at step S101B there is received at least one supplementary signal from one or more additional sensors and further at step S102B there is determined, based on the at least one supplementary signal, and the predefined reporting configuration whether to report the incontinence event.

In a further preferred embodiment, which may be combined or not with steps S101B, S102B, the method executes step S104 to communicate the reported incontinence event to an external device.

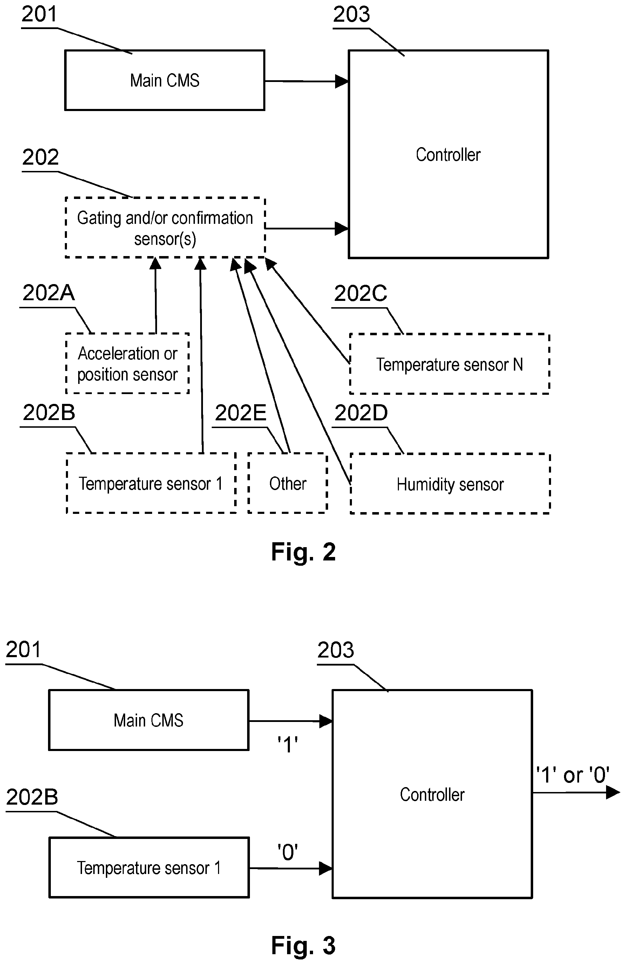

Fig. 2 presents a schematic drawing of the application of gating sensors according to the present disclosure. In order to reduce reporting of false-positive events, gating sensors may be used to identify events and circumstances, which could affect the interpretation of the main CMS measurements.

To this end the main CMS 201 reports its signals to a controller 203 that also optionally receives gating signals from one or more gating and/or confirmation sensors 202. Such gating sensors may be selected from a group comprising: a temperature signal 202B, 202C, a humidity signal 202D, an acceleration signal 202A, a gas presence signal 202E, a motion signal 202E, an electromagnetic signal 202E, a chemical composition detection signal 202E.

Such gating and/or confirmation sensor(s) 202 may additionally and/or alternatively comprise one or more confirmation sensor(s) in order to increase the confidence level of detection by the main CMS. The further confirmation sensors may also be one or more further CMS(s) falling under item 202E in Fig. 2.

The confirmation sensors are designed to detect incontinence events in parallel to the main CMS 201 as shown in Fig. 2, thereby providing enhanced confidence level in the reporting of the event detection.

All or some of the confirmation sensors can be physically the same as the gating sensor, but signals provided by the confirmation sensors may be analyzed using a different method, such that positive events are detected under a confirmation function and gating is performed under a gating function.

For example, confirmation sensors can include a humidity sensor and a temperature sensor, such that when a temperature increase is measured while a humidity decrease is also measured, an indication of a possible event is provided. This event of increased temperature in combination with decreased humidity can be indicative of an activation of super-absorbent material within the absorbent article, due to a urination event, wherein the urine provides an increase in temperature, while the activation of the super-absorbent material affects a reduction of humidity in the vicinity of the article.

Fig. 3 shows a schematic drawing of a use case of a main sensor and a temperature sensor according to the present invention. The figure presents the main CMS 201 operating together with the temperature gating sensor 202B wherein both sensors report signals to the controller 203.

In such a case if the temperature goes up, but not the capacitance, then maybe the resident has a fever or is sitting next to a furnace/radiator.

However, if the capacitance goes up, but not the temperature, then the resident may have sat in water or another source of non-internal liquid.

Such distinctive events may be reported accordingly.

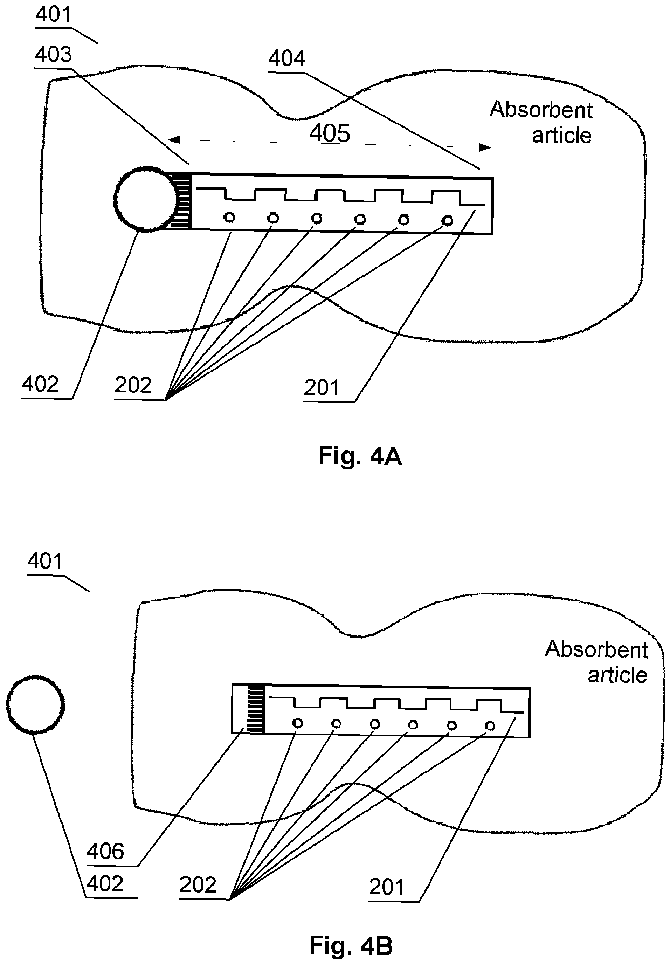

Figs. 4A and 4B illustrate an absorbent article comprising the system according to the present invention in two exemplary embodiments. In Fig. 4A an absorbent article 401 is equipped with a system according to the present invention where a strip-like or band-like article comprising respective sensors may be positioned on or in the absorbent article 401 by having its first end 403 positioned in proximity to the abdomen area (in particular the pelvic region) and having its second end 404 in proximity to the lumbar area whereas the length 405 of the strip/band runs along the crotch area.

Along the length 405 there may be positioned different gating and/or confirmation sensors 202 as well as the main CMS 201. The gating and/or confirmation sensors 202 may be spaced at regular intervals or be spaced at different distances from one another for example depending on different zones of the absorbent material 401.

Although a straight strip/band is shown in the figure, the strip/band may be shaped differently for example as an arc, as a wave or as a zig-zag arrangement.

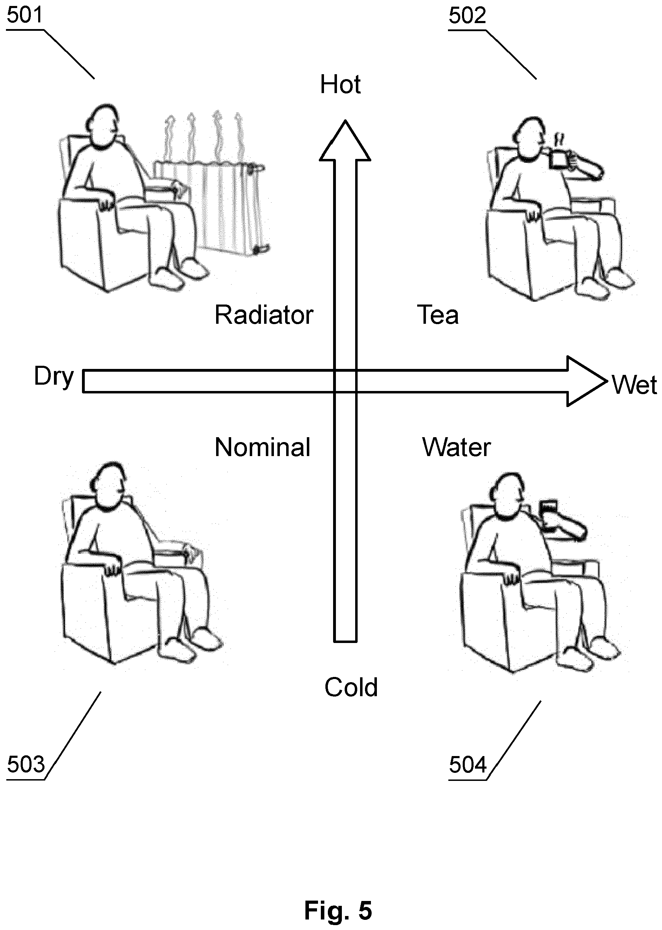

The controller 203 and other modules such as a battery and/or a communication module (shown in more details in Fig. 5) may be positioned at one end of the strip/band and preferably be protected by a fluid-tight casing 402. A connector may be present between the module 402 and the strip/band comprising sensors.

The module 402 may also comprise sensors, for example sensors such as an acceleration sensor or a motion sensor that are typically not present more than once.

Since the module 402 comprises sensors that are typically not present more than once such a module 402 may be, as shown in the embodiment of Fig. 4B, a separate module in communication with the strip/band which is equipped with modules 406 required for such a communication that are typical in the art.

When the module 402 is for example present in a wrist band of a user, use of additional sensors such as heart rate sensors is also possible as a gating sensor 202. The module 402 may be called a wearable or a clip.

As used herein, the term "wearable" 402 is a module that is configured to generate data from a suite of on-board sensors such as: moisture sensor, thermometer, motion sensor, heart rate sensor. This wearable 402 is connected to the absorbent article. The monitored absorbent article may also be called an incontinence material, ICM, or a brief that the subject or subjects are using for incontinence.

Fig. 5 illustrates a schematic drawing of combining data from sensors in two or more measurement dimensions. This provides improved triggering logic as well as non-medical insights into the residents' daily profiles. Four states of a resident are depicted in Fig. 5, wherein state 502 on the upper right concerns an event related to a hot and wet qualities such as hot tea or urine. The state 501 on the upper left concerns a state of sitting near a furnace/radiator (or otherwise a heat source) resulting in a dry and hot environment. The state 504 on the lower right concerns a state of dropping water (not warm liquid but relatively cold) on the absorbent article comprising the present system. Lastly, the state 503 on the lower left concerns a state of being relatively dry and at body temperature (or in proximity thereof) that denotes a nominal state where incontinence does not need to be reported.

Fig. 5 therefore identifies four conditions relevant to a use of the system according to the present disclosure. A client focused purpose for diagnosing these specific resident states would be to not to flag a medical event trigger when a medical event has not occurred, i.e., the person dropped/spilled tea on himself/herself, and therefore does not have a fever. Another purpose is preferably to provide insights into the data-driven conditions of different residents, such as their behavior during use of an absorbent article, which allows in turn to create residents' profiles.

A data focused purpose for testing these specific resident states is to improve the accuracy of certain medical triggering profiles. In the case of using only the main sensor to determine content of the absorbent article, there may be misclassified a resident's urination event and thus the fullness of their absorbent article. During urination, an absorbent article will approach full volume closer to the genitalia, and thus leave dry the region of the absorbent article that is closer to the abdomen (or at least with room for more liquid). If a resident drops/spills tea on their abdomen, the region of the absorbent article near their genitalia will continue to have space for more urine. Missing this evaluation (i.e., dropping tea) would flag the absorbent article as full before it is necessary to change it, thus reducing the efficiency of care givers using the present system.

Another example of a scenario to maximize accuracy would be to detect warming up of the absorbent article without detecting a corresponding introduction of liquid into the absorbent article. This would fulfill two purposes: one is to identify a non-urination event without triggering reporting of an incontinence event, i.e., a resident does not require attention. The other purpose would be to assess the thermometers' ability to measure the body temperature of the resident between urination events.

Regarding use of thermometers there are possible two approaches, one is to measure a resident's core temperature using the thermometers during a urination event; while the second is to measure the surface temperature without the need for a urination event. If the absorbent article's temperature is far above a human normal (i.e. sitting next to a furnace/radiator) it can also be determined that the source of excess heat is not near the genitalia, i.e. not caused by a urination event.

Combining sensors in just these two measurement dimensions provides improved triggering logic and provide non-medical insights into the residents' daily profiles.

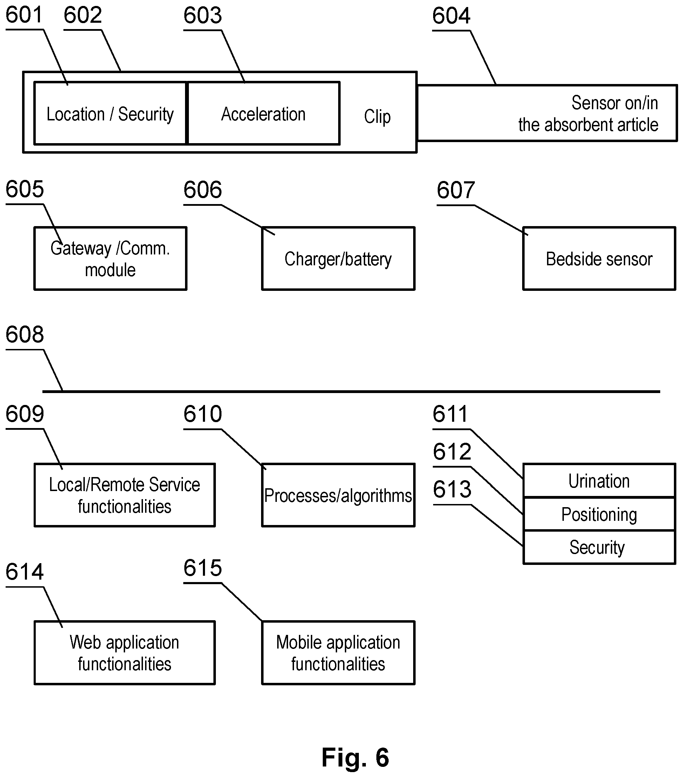

Fig. 6 shows a block diagram of the system according to the present invention. The figure shows, at a high abstraction level, modules that in a real system are connected either by wires or wirelessly. To this end the local part of the system comprises typical modules such as the controller (item 203 in Fig. 2), a memory, a data bus, a power source 606, a communication module 605 that may be wired (such as a USB port) or wireless such as Bluetooth LE, Wi-Fi, NFC, or the like.

The splitting line 608 logically splits physical features 601 to 607 from logical features 609 to 615 typically implemented by computer software.

A bedside sensor 607 may be present locally and configured to be an additional trigger option for a patient. Support for such a bedside sensor could optionally be provided by the present system.

The system including a server for analyzing sensor data, and a sensor assembly 601 to 604 is illustrated, including a clip 602 for communicating the signals of the sensors, as well as a sensor-strip 604, including a main CMS, as well as other secondary sensors, such as temperature sensors. The clip may also include sensors, such as volatile gas sensors, accelerometer(s) or positioning sensors, humidity sensors, and the like.

Sensors such as location and or security 601 sensor or acceleration sensor 603 (other sensors have been described with reference to Fig. 2) may be present in a wearable 402 as described with reference to Fig. 4A-B.

In turn the logical features 609 to 615 comprise main processing algorithms 610 as well as local/remote functions 609 that are related to reporting events. As mentioned above, some functions i.e. processing algorithms 610 may be implemented by remote devices that for example have greater processing power than the local system. For data processing efficiency some tasks may be executed remotely.

Configuration and/or management of the system may be effected by means of web application(s) 614 or mobile application(s) 615.

Memory of the system may store different data on different states of the system such as presence/absence of events related to urination 611, presence of events related to positioning and data related to positioning and/or motion as well as data related to security 613. Security measures may comprise data encryption as well as access control.

### Reference signs

- S101 to S104: Method steps
- 201: Main capacitive moisture sensor, CMS
- 202: Gating and/or confirmation sensor(s)
- 202A: Acceleration or position sensor
- 202B: Temperature sensor 1
- 202C: Temperature sensor N
- 202D: Humidity sensor
- 202E: Other sensor(s)
- 203: Controller
- 401: Absorbent article
- 402: Module
- 403: First end of the strip-like or band-like article
- 404: Second end of the strip-like or band-like article
- 405: Length of the strip-like or band-like article
- 406: Communication module
- 501: Dry and hot state
- 502: Wet and hot state
- 503: Dry and cold state
- 504: Wet and cold state
- 601: Location and or security sensor
- 602: Clip
- 603: Acceleration sensor
- 604: Sensor-strip
- 605: Communication module
- 606: Power source, charger/battery
- 607: Bedside sensor
- 608: Logical split between physical features and logical features
- 609: Local/remote functions
- 610: Processes / algorithms
- 611: State of urination
- 612: State of positioning
- 613: State of security
- 614: Web application(s) functions
- 615: Mobile application(s) functions

## Claims

1. A method for detecting an incontinence event in an absorbent article (401), the method comprising the steps of:
receiving (S101) a signal from a capacitive moisture sensor, CMS, (201) attached to the absorbent article (401), the signal being dependent on presence of liquid in the absorbent article;
determining (S102) based on the signal and a predefined reporting configuration comprising a reference value associated with the signal, whether to report an incontinence event; and
reporting (S103) an incontinence event based on the determination (S102).

2. The method according to claim 1, further comprising the steps of:
receiving (S101B) at least one supplementary signal from one or more additional sensors (202);
determining (S102B), based on the signal, the at least one supplementary signal, and the predefined reporting configuration further comprising a reference value associated with the at least one supplementary signal, whether to report the incontinence event.

3. The method according to claim 2, wherein the at least one supplementary signal comprises a capacitance measurement of the absorbent article (401) and/or wherein the at least one supplementary signal is selected from a group comprising: a temperature signal, a humidity signal, an acceleration signal, a gas presence signal, a motion signal, an electromagnetic signal, a chemical composition detection signal.

4. The method according to any of the preceding claims, wherein the predefined reporting configuration defines at least one reporting condition and wherein the incontinence event is reported when the at least one reporting condition has been met.

5. The method according to any of the preceding claims, wherein the predefined reporting configuration indicates a remaining capacity threshold of the absorbent article (401).

6. The method according to any of the preceding claims, wherein the CMS (201) is configured to detect the signal by performing a fringe capacitance measurement.

7. The method according to any of the preceding claims, wherein the CMS (201) is configured to detect the signal by detecting a frequency dependent capacitance at a plurality of frequencies.

8. The method according to claim 7, wherein the frequency dependent capacitance is detected at the plurality of frequencies in a range of 100 Hz to 100 kHz and/or in a range of 10 kHz to 100 kHz and/or in a range of 40 kHz to 80 kHz.

9. The method according to claim 7 or 8, wherein the determining (S102), whether to report the incontinence event, further comprises performing a cross-comparative and/or cross-correlative analysis of the capacitance detected at the plurality of frequencies.

10. The method according to any of the preceding claims further comprising a step of:
communicating (S104) the reported incontinence event to an external device (609).

11. A system for detecting incontinence events in an absorbent article (401), the system comprising:
a capacitive moisture sensor, CMS, (201) attached to the absorbent article (401), the CMS (201) being configured to induce an electrical stimulus signal to the absorbent article (401) and to determine a signal by a capacitance measurement, wherein the signal is dependent on a presence of liquid in the absorbent article (401); and
a controller (203) configured to receive a data signal from the CMS (201);
the controller (203) being further configured to execute the steps of the method according to claim 1.

12. The system according to claim 11, wherein the CMS (201) is configured to detect fringe capacitance.

13. The system according to claims 11 or 12, further comprising one or more additional sensors (202) configured to provide at least one supplementary signal;
wherein the controller (203) is further configured to execute the steps of the method according to claim 2.

14. The system according to any of claims 11 to 13, wherein the controller (203) is further configured to:
transmit data received from the CMS (201) and/or the additional sensors (202) to an external server for determining whether to report the incontinence event; and
receive from the external server a result of the determination.

15. The system according to any of the claims 10 to 14, wherein the one or more additional sensors (202) comprise:
gating sensors (202A to 202D), particularly selected from a group comprising: a temperature sensor, a humidity sensor, an acceleration sensor, a gas sensor, a motion sensor, an electromagnetic radiation sensor, a chemical composition detection sensor; and/or
at least one confirmation sensor, particularly at least one CMS attached to the absorbent article.
